# EUROPEAN PATENT SPECIFICATION

(11) **EP 0 336 318 B1**
(45) Date of publication and mention of the grant of the patent: **22.06.1994**
(21) Application number: 89105720.0
(22) Date of filing: 31.03.1989
(51) Int. Cl.: A61F 2/16

(54) **Intraocular lens with shape memory and its preparation**
Intraokulare Linse mit Formerinnerungsvermögen und deren Herstellung
Lentille intraoculaire avec mémoire de forme ainsi que sa fabrication

(30) Priority: 01.04.1988 CS 2261/88
(43) Date of publication of application: 11.10.1989
(73) Proprietor: CESKOSLOVENSKA AKADEMIE VED, Praha 1 (CS)
(72) Inventor: Sulc, Jiri, Dipl.-Ing., Praha 6 (CS); Krcova, Zuzana, Dipl.-Ing., Praha 7 (CS)
(74) Representative: Beetz & Partner Patentanwälte

(56) References cited:
- GB-A- 2 199 672
- US-A- 4 384 097
- US-A- 4 573 998
- US-A- 4 731 079

## Description

The invention relates to a hard intraocular lens with shape memory, which can be used for replacing the natural eye lens and is provided for insertion into the eye, i.e. into the anterior or posterior chamber or closely under the cornea, and methods for its preparation.

The lenses used so far for this purpose have a definite shape and physical state already before their surgical insertion into the eye. These lenses are made either from hard polymers of esters of methacrylic or acrylic acid with lower aliphatic alcohols, e.g. methyl methacrylate (MMA), or from soft hydrogels swollen in physiological saline to equilibrium before insertion into the eye. A relatively long incision is necessary in both cases in order to insert the lens into its place. Hard lenses cannot be deformed at all at applicable temperatures, and thus the insertion cannot be facilitated, while the possible temporary deformation of common hydrogel lenses on the basis of slightly crosslinked polymers, e.g. polymers of hydroxyethyl methacrylate (HEMA), is either limited or excluded due to their liability to break at too sharp bending. However, hard lenses increase the risk of injury of the sensitive eye tissues during insertion into the eye.

A substantial improvement represent intraocular lenses which are in an effectively deformed state before and during the operation thus enabling to reduce the length of the surgical incision to a minimum, and which acquire their definite, final shape in the correct position in the eye.

The intentional deformation consists, for example, in coiling or compressing the lens into a narrow rod or, at least, in a simple or multiple folding or rolling, in order to reduce the necessary length of the incision at least to a half.

These lenses are swollen prior to the operation in a swelling agent to such an extent that their glass-transition temperature T_{g} is between -5 and 45 °C. The lenses containing a reduced amount of swelling agent, so that T_{g} is in the above indicated region, are deformed in the relaxed state to a shape suitable for operation, for example, by coiling from both sides, and cooled below T_{g} to fix this deformation.

After the operation, which is considerably facilitated by this deformation, the lenses relax in the eye by post-swelling during warming to the temperature of the eye, and in this way acquire the desired original shape, elasticity and softness.

US-A-4 731 079 discloses an intraocular lens made of a polymeric material having a softening point in the range of body temperature. Prior to inserting into the eye, the lens is dimensionally reduced by compressing or actually extending at a temperature above the softening point, and the deformed state is frozen by cooling the lens below its softening temperature. The cooled, deformed lens is then inserted into the eye where it regains its original confirmation through the action of the body heat.

Since the rapidity of decoiling is predetermined, the surgeon has to work very quickly and with skill, and must not make any mistake. Moreover, the intraocular lens which floats in the body liquid responds to vibrations. A soft intraocular lens is more subject to such vibrations and transmits them to the haptics supporting the intraocular lens by the contact with the fibrous apparatus of the eye. This leads to a stress of the tissue in the contact regions and may cause gradual atrophy. A deformation of the optical part and defocussing of the optics may also occur as a consequence of vibrations of soft elastic intraocular lenses.

US-A-43 84 097 discloses extracameral contact lenses and contact shells consisting essentially of a copolymer of
(I) 40 to 90 % of methyl methacrylate and
(II) 10 to 60 % of methyl acrylate and/or esters of the formula 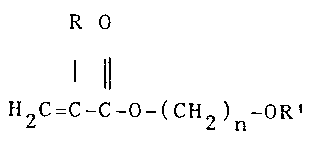 wherein
   - R =: H or CH₃,
   - R' =: H, CH₃ or CH₂H₅ and
   - n =: 1 or 2.

The glass-transition temperature T_{g} of these copolymers may be 40 to 70 °C.

This document does not deal with the specific problems involved with intracameral lenses and the specific problem of insertion of such lenses into the eye without suture.

It is the object of the present invention to provide a hard intraocular lens with a shape memory which is free from the shortcomings as discussed above, particularly vibration sensitivity and defocussing effects, and can be deformed before insertion into the eye without applying a swelling agent and without time limitation, and methods for its preparation.

The above object is achieved according to claims 1,5 and 11. The dependent claims relate to preferred embodiments.

The intraocular lens having a shape memory is characterized according to the invention in that it consists of a polymer, and particularly a copolymer, having a glass-transition temperature (T_{g}) of 40 to 60 °C, which is higher than the maximal eye temperature, thus the lens being still hard at maximum eye temperature.

According to a preferred embodiment, the intraocular lens, which preferably consists of a hydrophilic copolymer, is deformed into a shape suitable for insertion into the human eye, advantageously into a straight or bent rod with a diameter of 1 to 4 mm. With this lens, a suture-free insertion into the eye is possible.

The intraocular lens according to the invention preferably consists of a polymer and particularly of a copolymer of methyl methacrylate with butyl methacrylate, 2-hydroxyethyl-methacrylate and/or methacrylamide, and the like. The ratio of individual monomers is chosen in such a way that the glass-transition temperature is in the above-given range.

One of the methods for preparing the hard intraocular lens according to the invention is characterized by polymerizing a monomer or monomer mixture under such polymerization conditions that the resulting polymer/copolymer has a glass-transition temperature (T_{g}) of 40 to 60 °C.

A preferred method for preparing the hard intraocular lens according to the invention is based on the copolymerisation of a monomer mixture and is characterized in that the mass ratio of the individual comonomers is chosen such that the glass-transition temperature (T_{g}) of the resulting copolymer is 40 to 60 °C.

The shaping of the polymers/copolymers is done by polymerizing or copolymerizing the monomers in a suitable mold or by shape cutting of the obtained polymer or copolymer.

The lens according to the invention preferably consists of a copolymer of methyl methacrylate with butyl methacrylate, 2-hydroxyethyl methacrylate and/or methacrylamide.

The lens having the given T_{g} is heated at a temperature above T_{g}, e.g., at about 50 °C, deformed at this temperature, and cooled below T_{g} to fix the deformation. The lens deformed in this way remains in the deformed state after the surgical insertion into the eye. For relaxing the lens, the eye is rinsed with a suitable irrigation solution of a temperature above T_{g}, e.g., above 40 °C. First then it starts do decoil into its final shape and becomes hard again after the temperature decreases to 37 °C, i.e. below T_{g}.

This hard intraocular lens is not affected by vibrations in the eye, and a defocussing of optics does not occur. The intraocular lens according to the invention combines the advantages of soft intraocular lenses with respect to the possible deformation for the purpose of operation and that of hard intraocular lenses, made for example from methyl methacrylate, with respect to the behavior in the eye.

The invention is further explained with reference to an example.

### Example

A mixture of 40 parts by mass of methyl methacrylate and 60 parts by mass of 2-hydroxyethyl methacrylate (HEMA) was placed in an ampoule and added with 0,05 % of azo-bis(isobutyronitrile); the ampoule was then purged with argon and sealed. Thereafter, it was heated in a water bath at 60 °C for 48 h and at 80 °C for further 24 h.

An intraocular lens was made from the plug removed from the ampoule by turning. The lens was swollen in physiological saline, deformed at 50 °C into a rod-like shape with a diameter of 2 mm, and cooled down. The deformed lens was then inserted into an eye through a small 3 mm incision. After elevating the temperature by rinsing with a solution heated to 48 °C, the lens softened and acquired its original shape. After the temperature decreased to 37 °C, i.e. to the human body temperature, the lens became hard again and behaved as a lens made from poly(methyl methacrylate) (PMMA).

## Claims

1. Intraocular lens consisting of a polymer and having a shape memory, characterized in that the polymer has a glass-transition temperature (T_{g}) of 40 to 60 °C and is still hard at maximum eye temperature.

2. The lens according to claim 1, characterized in that it consists of a copolymer, preferably a hydrophilic copolymer.

3. The lens according to claim 1 or 2, characterized in that it consists of a copolymer of methyl methacrylate with butyl methacrylate, 2-hydroxyethyl methacrylate and/or methacrylamide.

4. The lens according to one of claims 1 to 3, characterized in that it is present in a deformed state having the shape of a straight or bent rod of a diameter of 1 to 4 mm.

5. A method for preparing the intraocular lens according to one of claims 1 to 4, characterized by polymerizing a monomer or monomer mixture under such polymerization conditions that the glass-transition temperature (T_{g}) of the resulting polymer is 40 to 60 °C.

6. The method according to claim 5 with copolymerization of a monomer mixture, characterized in that the mass ratio of the individual comonomers is chosen such that the glass-transition temperature (T_{g}) of the resulting copolymer is 40 to 60 °C.

7. The method according to claim 5 or 6, characterized in that shaping is done by polymerizing or copolymerizing in a suitable mold or by shape cutting of the obtained polymer or copolymer.

8. The method according to one of claims 5 to 7, characterized in that methyl methacrylate, butyl methacrylate, 2-hydroxyethyl methacrylate and/or methacrylamide are used as monomers/comonomers.

9. The method according to one of claims 5 to 8, characterized in that after polymerization/copolymerization, the lens is deformed into the desired shape at a temperature above its glass-transition temperature (T_{g}), and its deformation state is fixed by cooling down with retaining the shape to a temperature below T_{g}.

10. The method according to claim 9, characterized in that the lens is deformed into the shape of a straight or bent rod of a diameter of 1 to 4 mm.

11. Use of polymers having a glass-transition temperature (T_{g}) of 40 to 60 °C, being higher than the maximum eye temperature, which are deformable at temperatures above T_{g}, and which are hard even at maximum eye temperature, for preparing intraocular lenses for suture-free insertion into the human eye.

## Patentansprüche

1. Intraokulare Linse, die aus einem Polymer besteht und über ein Formgedächtnis verfügt,
dadurch **gekennzeichnet,** daß
das Polymer eine Glasübergangstemperatur (T_{g}) von 40 bis 60 °C aufweist und bei der höchsten Augentemperatur immer noch hart ist.

2. Intraokulare Linse nach Anspruch 1,
dadurch gekennzeichnet, daß
sie aus einem Copolymer, vorzugsweise einem hydrophilen Copolymer, besteht.

3. Intraokulare Linse nach Anspruch 1 oder 2,
dadurch gekennzeichnet, daß
sie aus einem Copolymer von Methylmethacrylat mit Butylmethacrylat, 2-Hydroxyethylmethacrylat und/oder Methacrylamid besteht.

4. Intraokulare Linse nach einem der Ansprüche 1 bis 3,
dadurch gekennzeichnet, daß
sie in einem deformierten Zustand vorliegt, in dem sie die Form eines geraden oder gebogenen Stabes mit einem Durchmesser von 1 bis 4 mm hat.

5. Verfahren zur Herstellung der intraokularen Linse nach einem der Ansprüche 1 bis 4,
gekennzeichnet durch
Polymerisation eines Monomers oder eines Monomergemischs unter solchen Polymerisationsbedingungen, daß die Glasübergangstemperatur (T_{g}) des entstehenden Polymers 40 bis 60 °C beträgt.

6. Verfahren nach Anspruch 5 unter Copolymerisation eines Monomergemischs,
dadurch gekennzeichnet, daß
das Massenverhältnis der einzelnen Comonomeren so gewählt wird, daß die Glasübergangstemperatur des entstehenden Copolymers 40 bis 60 °C beträgt.

7. Verfahren nach Anspruch 5 oder 6,d
dadurch gekennzeichnet, daß
die Formgebung durch Polymerisation oder Copolymerisation in einer geeigneten Gießform oder durch Schneiden des erhaltenen Polymers oder Copolymers zur entsprechenden Form geschieht.

8. Verfahren nach einem der Ansprüche 5 bis 7,
dadurch gekennzeichnet, daß
Methylmethacrylat, Butylmethacrylat, 2-Hydroxyethylmethacrylat und/oder Methacrylamid als Monomere/Comonomere verwendet werden.

9. Verfahren nach einem der Ansprüche 5 bis 8,
dadurch gekennzeichnet, daß
nach der Polymerisation/copolymerisation die Linse bei einer Temperatur oberhalb ihrer Glasübergangstemperatur (T_{g}) zur gewünschten Form deformiert wird und ihr deformierter Zustand durch Abkühlen auf eine Temperatur unter T_{g} unter Beibehaltung der Form fixiert wird.

10. Verfahren nach Anspruch 9,
dadurch gekennzeichnet, daß
die Linse zur Form eines geraden oder gebogenen Stabes mit einem Durchmesser von 1 bis 4 mm deformiert wird.

11. Verwendung von Polymeren mit einer Glasübergangstemperatur (T_{g}) von 40 bis 60 °C, die höher ist als die höchste Augentemperatur, die bei Temperaturen oberhalb T_{g} deformierbar sind und die selbst bei der höchsten Augentemperatur noch hart sind, zur Herstellung von intraokularen Linsen zum Einsetzen in das menschliche Auge ohne chirurgische Naht.

## Revendications

1. Lentille intraoculaire constituée d'un polymère et ayant me mémoire de forme, caractérisée en ce que le polymère a une température de transition au verte (T_{g}) de 40 à 60°C et est encore dure à la température maximale de l'oeil.

2. La lentille selon la revendication 1, caractérisée en ce qu'elle est constituée d'un copolymère, de préférence un copolymère hydrophile.

3. La lentille selon la revendication 1 ou 2, caractérisée en ce qu'elle est constituée d'un copolymère de méthacrylate méthylique avec du méthacrylate butylique, du 2-hydroxyéthyl méthacrylate et/ou du méthacrylamide.

4. La lentille selon une des revendications 1 à 3, caractérisée en ce qu'elle est présente à un état déformé ayant la forme d'un bâtonnet droit ou courbe d'un diamètre de 1 à 4 mm.

5. Un procédé pour préparer la lentille intraoculaire selon une des revendications 1 à 4, caractérisé par la polymérisation d'un monomère ou d'un mélange de monomères dans des conditions de polymérisation telles que la température de transition au verte (T_{g}) du polymère résultant est de 40 à 60°C.

6. Le procédé selon la revendication 5 avec copolymérisation d'un mélange de monomères, caractérisé en ce que le rapport des masses des comonomères particuliers est choisi de telle sorte que la température de transition au verte (T_{g}) du copolymère résultant est de 40 à 60°C.

7. Le procédé selon la revendication 5 ou 6, caractérisé en ce que la mise en forme est faite par polymérisation ou copolymérisation dans un moule adéquat ou par découpage de forme du polymère ou du copolymère obtenu.

8. Le procédé selon une des revendications 5 à 7, caractérisé en ce que le méthacrylate méthylique , le méthacrylate butylique, le 2-hydroxyéthyl méthacrylate et/ou le méthacrylamide sont utilisés comme monomères/comonomères.

9. Le procédé selon une des revendications 5 à 8, caractérisé en ce qu'après polymérisation/copolymérisation, la lentille est déformée sous la forme désirée à une température au-dessus de sa température de transition au verre (T_{g}), et son état de déformation est fixé par refroidissement avec maintien de la forme à une température en dessous de T_{g}.

10. Le procédé selon la revendication 9, caractérisé en ce que la lentille est déformée sous la forme d'un bâtonnet droit ou courbe d'un diamètre de 1 à 4 mm.

11. Utilisation des polymères ayant une température de transition au verte (T_{g}) de 40 à 60°C, étant supérieure à la température maximale de l'oeil, qui sont déformables aux températures au-dessus de T_{g}, et qui sont durs même à la température maximale de l'oeil, pour la préparation des lentilles intraoculaires pour l'insertion sans suture dans l'oeil humain.
